(19) Europäisches Patentamt / European Patent Office / Office européen des brevets

(11) **EP 2 648 013 A2**

(12) **EUROPEAN PATENT APPLICATION**

(43) Date of publication:
**09.10.2013 Bulletin 2013/41**

(51) Int Cl.:
*G01R 33/3815* (2006.01)　　　*H01F 6/00* (2006.01)

(21) Application number: **12189443.0**

(22) Date of filing: **22.10.2012**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME** | (72) Inventor: **Milward, Stephen R.**<br>**Witney**<br>**Oxon OX29 6PZ (GB)** |
| (30) Priority: **12.03.2012 KR 20120025224** | (74) Representative: **Grootscholten, Johannes A.M. et al**<br>**Arnold & Siedsma**<br>**Sweelinckplein 1**<br>**2517 GK Den Haag (NL)** |
| (71) Applicant: **Samsung Electronics Co., Ltd**<br>**Gyeonggi-do 443-742 (KR)** | |

(54) **Persistent switch control system, superconducting magnet apparatus employing the same, and method of controlling persistent switch**

(57)　A persistent switch control system, a superconducting magnet apparatus employing the persistent switch control system, and a method of controlling a persistent switch. The persistent switch control system includes: a persistent switch for switching between an open state and a closed state of a superconducting coil; and a persistent switch controller for controlling the persistent switch, wherein, during a charging mode, a resistance state of the persistent switch is maintained by a ramp heat load that is generated by a ramp voltage applied to the persistent switch.

FIG. 1

EP 2 648 013 A2

**Description**

**CROSS-REFERENCE TO RELATED PATENT APPLICATION**

This application claims the benefit of Korean Patent Application No. 10- 2012- 0025224, filed on March 12, 2012, in the Korean Intellectual Property Office, the disclosure of which is incorporated herein in its entirety by reference.

**BACKGROUND**

**1. Field**

Systems, apparatuses, and methods consistent with exemplary embodiments relate to a persistent switch control system, a superconducting magnet apparatus employing the persistent switch control system, and a method of controlling a persistent switch.

**2. Description of the Related Art**

A superconducting apparatus such as a magnetic resonance imaging (MRI) apparatus or a nuclear magnetic resonance (NMR) apparatus uses a superconducting magnet. The superconducting magnet operates when a current is applied to a superconducting coil cooled at an ultralow temperature, such as 4.2K, so that a superconducting phenomenon is generated. The superconducting magnet operates often in a persistent mode and includes a persistent switch to enter the persistent mode. The persistent mode is for allowing a current to flow through a closed loop in a superconducting magnet, and the persistent switch is used to block a current flow to a current lead between a superconducting magnet at a low temperature and a magnet power source at a normal temperature. Such persistent switch provides high field stability in application devices such as NMR apparatuses and MRI apparatuses, and can reduce a low temperature heat load for increasing a liquid helium retention time of a cryostat.
Generally, the persistent switch includes a superconducting wire and a switch heater. As heat is applied from the switch heater to the superconducting wire, the superconducting wire has resistivity since the temperature thereof rises above a superconducting transition temperature. When the persistent switch is designed appropriately, the persistent switch has a relatively high resistance. A high resistance of the persistent switch in an open state allows the superconducting magnet to be charged. The conducting wire is cooled and restores superconductivity when the charge is completed, and thus, the persistent switch is closed.

**SUMMARY**

Applying heat to a persistent switch to open it causes a heat load on a cooling system of a superconducting magnet apparatus. For example, applying heat to a persistent switch to open it causes consumption of liquid helium. Furthermore, consumption of liquid helium is additionally increased due to heat dispersed from the persistent switch, which is caused by a ramp voltage that is applied to the persistent switch when the superconducting magnet is charged.
An aspect of an exemplary embodiment provides a persistent switch control system for reducing a heat load that is generated when a superconducting magnet is charged.
An aspect of an exemplary embodiment also provides a superconducting magnet apparatus employing the persistent switch control system.
Another aspect of an exemplary embodiment also provides a method of controlling a persistent switch.
According to an aspect of an exemplary embodiment, there is provided a persistent switch control system including: a persistent switch which switches between an open state and a closed state of a superconducting coil; and a persistent switch controller which controls the persistent switch, wherein, during a charging mode, a resistance state of the persistent switch is maintained by a ramp heat load that is generated by a ramp voltage applied to the persistent switch.
The persistent switch may include: a superconducting wire which constitutes a portion of a superconducting coil; and a switch heater which applies heat to the superconducting wire, wherein the persistent switch controller may supply a power supply voltage to the switch heater when the charging mode starts and may block the power supply voltage supplied to the switch heater when a current is supplied to the superconducting coil.
In the charging mode, a ramping rate of the current that is supplied to the superconducting coil may be set so that the ramp heat load, which is generated by the ramp voltage applied between ends of the superconducting wire, maintains a resistance state of the superconducting wire.
The supply of current to the superconducting coil may be stopped when the current that is supplied to the superconducting coil reaches a target current. The current that is supplied to the superconducting coil may be adjusted when it is substantially close to the target current. The persistent switch controller may turn on the switch heater before adjusting the current that is supplied to the superconducting coil, and may turn off the switch heater after adjusting the current that is supplied to the superconducting coil.

According to another aspect of an exemplary embodiment, there is provided a superconducting magnet apparatus including: a superconducting coil; a power source for a superconducting coil, which supplies a current to the superconducting coil; and a persistent switch control system, which controls an open state and a closed state of the superconducting coil. The persistent switch control system comprises: a persistent switch which switches between the open state and the closed state of the superconducting coil; and a persistent switch controller which controls the persistent switch, wherein, during a charging mode, a resistance state of the persistent switch is maintained by a ramp heat load that is generated by a ramp voltage applied to the persistent switch.

The superconducting magnet apparatus may be a magnetic resonance imaging (MRI) apparatus, a nuclear magnetic resonance (NMR) apparatus, or a superconducting magnet apparatus for a maglev car.

According to another aspect of an exemplary embodiment, there is provided a method of controlling a persistent switch for switching between an open state and a closed state of a superconducting coil, the method including maintaining a resistance state of the persistent switch by a ramp heat load that is generated by a ramp voltage that is applied to the persistent switch during a charging mode.

The persistent switch control system, the superconducting magnet apparatus employing the persistent switch control system, and the method of controlling a persistent switch may reduce a heat load, which is separately applied from the outside for operation of the persistent switch, by using the persistent switch's own ramp heat load in a charging mode.

## BRIEF DESCRIPTION OF THE DRAWINGS

The above and other aspects of exemplary embodiments will become more apparent with reference to the attached drawings in which:

FIG. 1 schematically illustrates a superconducting magnet apparatus employing a persistent switch control system, according to an exemplary embodiment;
FIG. 2 is a flowchart illustrating a method of controlling a persistent switch, according to an exemplary embodiment; and
FIG. 3 is a flowchart illustrating an example of an operation of minutely adjusting a current in the method of FIG. 2.

## DETAILED DESCRIPTION OF EXEMPLARY EMBODIMENTS

Hereinafter, exemplary embodiments will be described in detail with reference to the attached drawings. The same reference numerals in the drawings denote the same element. In the drawings, the thicknesses of layers and regions are exaggerated for clarity.

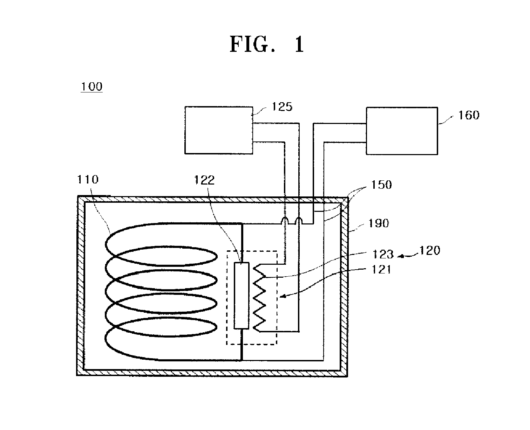

FIG. 1 schematically illustrates a persistent switch control system 120 according to an exemplary embodiment and a superconducting magnet apparatus 100 employing the persistent switch control system 120.

Referring to FIG. 1, the superconducting magnet apparatus 100 includes a superconducting coil 110, a current lead 150 that transmits a current to the superconducting coil 110, a power source 160 for a superconducting magnet, which supplies a current to the superconducting coil 110 through the current lead 150, and a cryostat 190 that maintains the temperature of the superconducting coil 110 in an ultralow temperature state. In addition, the superconducting magnet apparatus 100 includes a persistent switch control system 120. Furthermore, although not illustrated, the superconducting magnet apparatus 100 may further include a cooler that cools the superconducting coil 110 and maintains an ultralow temperature state of the cryostat 190.

The persistent switch control system 120 includes a persistent switch 121 that is disposed at one side of the superconducting coil 110 and a persistent switch controller 125 that is disposed outside the cryostat 190 and controls the persistent switch 121.

The persistent switch 121 switches between an open state of the superconducting coil 110 and a closed state thereof. That is, the state of the persistent switch 121 may become a resistance state and thereby allow the superconducting coil 110 to be in the open state, and may become a superconducting state and thereby allow the superconducting coil 110 to be in the closed state. For this, the persistent switch 121 includes a superconducting wire 122 and a switch heater 123 that is disclosed adjacent to the superconducting wire 122 and may apply heat to the superconducting wire 122. It may be understood that the superconducting wire 122 is a portion of the superconducting coil 110 in a persistent mode. From the viewpoint of the power source 160 for a superconducting magnet, the superconducting wire 122 and the superconducting coil 110 are connected to each other in parallel. The superconducting wire 122 may be formed of a non-inductive coil to have a minimum inductance. The superconducting wire 122 is designed to have a ramp heat load that may maintain a temperature for maintaining the resistance state (that is, a state in which the superconducting state is broken). That is, the superconducting wire 122 is designed so that a resistance value thereof in the resistance state has a predetermined ramp heat load. The ramp heat load will be described below.

The persistent switch controller 125 may control the persistent switch 121 by controlling a power supply that is applied to the switch heater 123. In addition, the persistent switch 121 may be controlled by the ramp heat load also in the state where the switch heater 123 has been turned off, and the ramp heat load may be controlled by adjusting a ramping rate of a current that is supplied from the power source 160 for a superconducting magnet.

The superconducting coil 110 may be electrically connected to the power source 160 for a superconducting magnet, which is disposed outside the cryostat 190, through the current lead 150, and thus may receive a current from the power source 160 in a charging mode. In the superconducting magnet apparatus 100, a current supply to the superconducting coil 110 is blocked in the persistent mode, and in some cases, the current lead 150 may have a detachable and attachable structure.

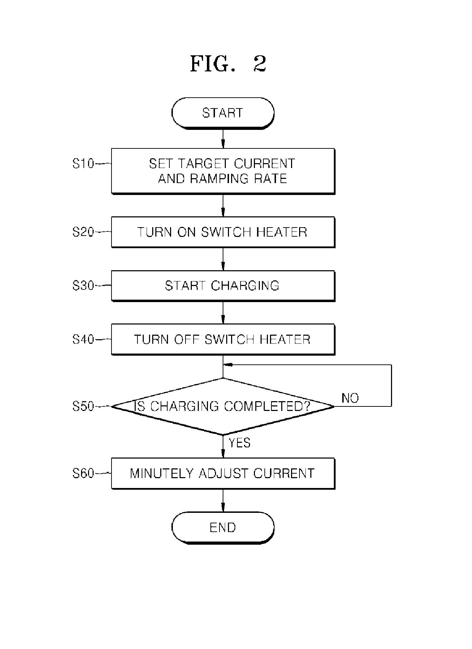

FIG. 2 is a flowchart illustrating a method of controlling a persistent switch in the superconducting magnet apparatus 100, according to an exemplary embodiment. The method of controlling a persistent switch will be described with reference to FIGS. 1 and 2.

The superconducting coil 110 is cooled in the superconducting state during operation of the superconducting magnet apparatus 100. The superconducting magnet apparatus 100 may have a charging mode in which a current is supplied to the superconducting coil 110 to charge it and a persistent mode in which a current flows in a closed circuit of the superconducting coil 110.

Prior to start of the charging mode, a target current and a ramping rate are set (operation S10). The target current is the amount of current that is used for determining whether charging has been completed. The ramping rate is a change of current via time, in which the current is gradually increased until the target current is reached. The ramping rate is set in consideration of a load that is generated by the inductance of the superconducting coil 110, and is set so that the superconducting wire 122 may maintain the resistance state by using a ramp heat load thereof in the charging mode.

Next, when the charging mode starts, the persistent switch controller 125 applies heat to the superconducting wire 122 to break the superconducting state thereof, by supplying a current to the switch heater 123, and changes the state of the superconducting wire 122 from the superconducting state to the resistance state (operation S20) . For example, the amount $H_r$ of heat per hour, which is generated in the switch heater 123, may be obtained according to equation 1 below.

$$H_r = I_h^2 \cdot R_h \qquad (1)$$

$I_r$ denotes a current supplied to the switch heater 123, and $R_h$ denotes a resistance value of the switch heater 123. For example, if $R_h$ is 100$\Omega$ and $I_h$ is 30mA, the amount $H_r$ of heat per hour, which is generated in the switch heater 123, is 90mW, and the generated heat may increase the temperature of the superconducting wire 122 to a temperature over 10K by heating the superconducting wire 122. Thus, the state of the superconducting wire 122 may be changed from the superconducting state to the resistance state. If the state of the superconducting wire 122 is changed from the superconducting state to the resistance state, it may be understood that the persistent switch 121 is substantially in an open state while the superconducting coil 110 is in the superconducting state.

If the state of the persistent switch 121 is changed to the open state, the power source 160 for a superconducting magnet supplies a current to the superconducting coil 110 through the current lead 150 (operation S30). Since the superconducting wire 122 is in the resistance state in the charging mode, most of the current flows to the superconducting coil 110 that substantially has no resistance, and thus, the superconducting coil 110 is charged. In this case, the power source 160 for a superconducting magnet slowly increases a current (that is, ramps up a current), which is supplied to the superconducting coil 110, in consideration of the inductance of the superconducting coil 110. If a voltage $V_r$ is applied through the current lead 150 from the power source 160 for a superconducting magnet, the increase amount dI/dt of current that is supplied to the superconducting coil 110 may be obtained by the following equation 2.

$$\frac{dI}{dt} = \frac{V_r}{L} \qquad (2)$$

L denotes the inductance of the superconducting coil 110. The increase amount dI/dt of current is referred to as a ramping rate, and the voltage $V_r$ that is applied to the superconducting coil 110 is referred to as a ramp voltage.

Referring to equation 2, it may be understood that a current flowing through the superconducting coil 110 increases by the ramping rate Vr/L if the ramp voltage $V_r$ is applied to the superconducting coil 110.

Since the superconducting wire 122 is connected in parallel to the superconducting coil 110, the ramp voltage $V_r$ supplied from the power source 160 for a superconducting magnet is applied also between ends of the superconducting wire 122. Since the superconducting wire 122 is in the resistance state in the charging mode, the superconducting wire 122 generates Joule's heat due to the ramp voltage $V_r$. A heat load $H_r$ of the superconducting wire 122 (hereinafter, referred to as a ramp heat load), which is generated due to the ramp voltage $V_r$, may be obtained according to equation 3 below.

$$H_r = \frac{V_r^2}{R_s}$$

(3)

$R_s$ denotes the resistance of the superconducting wire 122 in the resistance state.

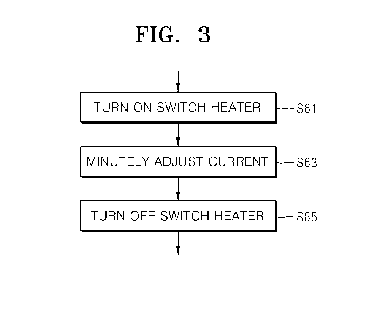

Referring to FIG. 3, in the resistance state, the superconducting wire 122 has a ramp heat load $H_r$ that is proportional to the square of the ramp voltage $V_r$ and is inversely proportional to the resistance $R_s$ of the superconducting wire 122. Conventional superconducting magnet apparatuses minimize a heat load of a persistent switch by enlarging the resistance of a superconducting wire of the persistent switch in the resistance state. However, since the superconducting magnet apparatus 100 according to the current exemplary embodiment uses heat that is generated in the resistance state of the superconducting wire 122, the resistance $R_s$ of the superconducting wire 122 is set to a small value such that the ramp heat load $H_r$ has a heat load value so that the superconducting wire 122 may be maintained in the resistance state when the switch heater 123 has been turned off. The heat load value to maintain the superconducting wire 122 in the resistance state may be changed according to a thermal environment of the superconducting wire 122. For example, if the resistance $R_s$ of the superconducting wire 122 is 500Ω and the ramp voltage $V_r$ is 4 V, the ramp heat load $H_r$ becomes 32mW, and the ramp heat load $H_r$ of 32mW may maintain the superconducting wire 122 in the resistance state when the switch heater 123 has been turned off.

When or immediately after the power source 160 for a superconducting magnet applies the ramp voltage $V_r$ to the superconducting coil 110, the persistent switch controller 125 blocks a current, which is applied to the switch heater 123, to change the state of the switch heater 123 to a turn-off state (operation of S40). Since the resistance and the ramping rate (or the ramping voltage) of the superconducting wire 122 according to the current exemplary embodiment is designed to have a ramp heat load $H_r$ so that the resistance state may be maintained in the charging mode, the superconducting wire 122 may maintain the resistance state due to heat, which is generated by the superconducting wire 122 itself, although the state of the switch heater 123 is changed to the turn-off state. Thus, since the persistent switch 121 is still in the open state, the superconducting coil 110 is charged by a current that is supplied from the power source 160 for a superconducting magnet.

Next, after a predetermined time has elapsed, the power source 160 for a superconducting magnet determines whether the charging of the superconducting coil 110 has been completed (operation S50) . For example, it is possible to determine whether the charging has been completed by determining whether a current that is supplied from the power source 160 for a superconducting magnet has reached a  target current. Until a current that is supplied from the power source 160 for a superconducting magnet is close to the target current, the turn- off state of the switch heater 123 and the charging state are maintained. In this case, a heat load that is generated in the switch heater 123 may be minimized since the switch heater 123 is in the turn- off state, and thus, a heat load that is applied to the cryostat 190 may be reduced.

If a current that is supplied from the power source 160 for a superconducting magnet reaches a target current, the power source 160 for a superconducting magnet stops the supply of current. A ramp heat load that is generated in the superconducting wire 122 is also reduced when the supply of current from the power source 160 is stopped, and thus, the superconducting wire 122 may be automatically cooled and then may return to the superconducting state. If the superconducting wire 122 returns to the superconducting state, the persistent switch 121 is closed and a current flowing through the superconducting coil 110 is turned toward the persistent switch 121 constituting a closed loop, and thus, the charging mode is ended.

Operation S60 of minutely adjusting a current may be further performed in the operation of stopping the supply of current from the power source 160 for a superconducting magnet. FIG. 3 illustrates an example of operation S60 of minutely adjusting a current. Referring to FIG. 3, if a current that is supplied from the power source 160 for a superconducting magnet is close to the target current, the power source 160 minutely adjusts the current to make the current reach the target current and then stops the supply of current if the current reaches the target current (operation S63). Since a change of the current in operation S63 may be relatively large or relatively small at need, the resistance state of the superconducting wire 122 may not be maintained by a ramp heat load alone that is generated in the superconducting wire 122. Thus, by further performing operation S61 of turning on the switch heater 123 prior to operation S63 of minutely adjusting a current and performing operation S65 of turning off the switch heater 125 again if operation S63 of minutely adjusting a current is completed, the persistent switch 121 may stably maintain an open state in operation S63 of minutely adjusting a current. If the supply of current from the power source 160 for a superconducting magnet is stopped and the switch heater 123 is turned off again, the persistent switch 121 is closed and a current flowing through the superconducting coil 110 is turned toward the persistent switch 121 constituting a closed loop, and thus, the charging mode is ended.

Since, as stated above, the superconducting magnet apparatus 100 according to the current exemplary embodiment places the switch heater 123 in the turn-off state except in the charging process and the minute current adjustment process, the superconducting magnet apparatus 100 may minimize a heat load that is generated in the switch heater 123, may reduce a heat load that is applied to a cooler of the superconducting magnet apparatus 100, and may improve heat efficiency.

The superconducting magnet apparatus 100 according to the current exemplary embodiment may be a magnetic resonance imaging (MRI) apparatus, a nuclear magnetic resonance (NMR) apparatus, a superconducting magnet apparatus for a maglev car, or the like. For example, if the superconducting magnet apparatus 100 is an MRI apparatus, the superconducting magnet apparatus 100 may further include a gradient coil or a radio frequency (RF) coil.

While exemplary embodiments have been particularly shown and described, it will be understood by those of ordinary skill in the art that various changes in form and details may be made therein without departing from the spirit and scope of the present invention as defined by the following claims.

## Claims

1. A persistent switch control system comprising:

   a persistent switch which switches between an open state and a closed state of a superconducting coil; and
   a persistent switch controller which controls the persistent switch,
   wherein, during a charging mode, a resistance state of the persistent switch is maintained by a ramp heat load that is generated by a ramp voltage applied to the persistent switch.

2. The persistent switch control system of claim 1, wherein the persistent switch comprises:

   a superconducting wire which constitutes a portion of a superconducting coil; and
   a switch heater which applies heat to the superconducting wire,
   wherein the persistent switch controller supplies a power supply voltage to the switch heater when the charging mode starts and blocks the power supply voltage supplied to the switch heater when a current is supplied to the superconducting coil.

3. The persistent switch control system of claim 2, wherein, in the charging mode, a ramping rate of the current that is supplied to the superconducting coil is set so that the ramp heat load, which is generated by the ramp voltage applied between ends of the superconducting wire, maintains a resistance state of the superconducting wire.

4. The persistent switch control system of claim 2 or 3, wherein the supply of current to the superconducting coil is stopped when the current that is supplied to the superconducting coil reaches a target current.

5. The persistent switch control system of claim 4, wherein the current that is supplied to the superconducting coil is adjusted when it is substantially close to the target current.

6. The persistent switch control system of claim 5, wherein the persistent switch controller turns on the switch heater before adjusting the current that is supplied to the superconducting coil, and turns off the switch heater after adjusting the current that is supplied to the superconducting coil.

7. A superconducting magnet apparatus comprising:

   a superconducting coil;
   a power source for the superconducting coil, which supplies a current to the superconducting coil; and

a persistent switch control system of any one of claims 1 to 6, which controls an open state and a closed state of the superconducting coil.

8. The superconducting magnet apparatus of claim 7, wherein the superconducting magnet apparatus is a magnetic resonance imaging (MRI) apparatus, a nuclear magnetic resonance (NMR) apparatus, or a superconducting magnet apparatus for a maglev car.

9. A method of controlling a persistent switch for switching between an open state and a closed state of a superconducting coil, the method comprising:

maintaining a resistance state of the persistent switch by a ramp heat load that is generated by a ramp voltage that is applied to the persistent switch during a charging mode.

10. The method of claim 9, wherein the persistent switch comprises a superconducting wire which constitutes a portion of the superconducting coil and a switch heater for applying heat to the superconducting wire, and
the method further comprising supplying a power supply voltage to the switch heater when the charging mode starts and blocking the power supply voltage which is supplied to the switch heater, when a current is supplied to the superconducting coil.

11. The method of claim 10, further comprising setting a ramping rate of the current that is supplied to the superconducting coil,
wherein the ramping rate is set so that the ramp heat load, which is generated by the ramp voltage that is applied between both ends of the superconducting wire, maintains a resistance state of the superconducting wire.

12. The method of claim 10 or 11, further comprising stopping the supply of current to the superconducting coil when the current that is supplied to the superconducting coil reaches a target current.

13. The method of claim 12, further comprising adjusting the current which is supplied to the superconducting coil, when the current that is supplied to the superconducting coil is substantially close to the target current.

14. The method of claim 13, wherein the adjusting of the current comprises:

turning on the switch heater before adjusting the current that is supplied to the superconducting coil, and
turning off the switch heater after adjusting the current that is supplied to the superconducting coil.

FIG. 1

## FIG. 2

START

S10 — SET TARGET CURRENT
AND RAMPING RATE

S20 — TURN ON SWITCH HEATER

S30 — START CHARGING

S40 — TURN OFF SWITCH HEATER

S50 — IS CHARGING COMPLETED? —— NO

YES

S60 — MINUTELY ADJUST CURRENT

END

FIG. 3

| | |
|---|---|
| TURN ON SWITCH HEATER | S61 |
| MINUTELY ADJUST CURRENT | S63 |
| TURN OFF SWITCH HEATER | S65 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020120025224